(19) Europäisches Patentamt
European Patent Office
Office européen des brevets

(11) Veröffentlichungsnummer: 0 320 775
A2

(12) **EUROPÄISCHE PATENTANMELDUNG**

(21) Anmeldenummer: 88120445.7

(22) Anmeldetag: 07.12.88

(51) Int. Cl.4: C07D 239/56 , A01N 43/54

(30) Priorität: 18.12.87 DE 3742970

(43) Veröffentlichungstag der Anmeldung: 21.06.89 Patentblatt 89/25

(84) Benannte Vertragsstaaten: BE CH DE FR GB IT LI NL SE

(71) Anmelder: BAYER AG
D-5090 Leverkusen 1 Bayerwerk(DE)

(72) Erfinder: Steinbeck, Karl, Dr.
12409 Overbrookroad
Leawood Kansas 66209(US)
Erfinder: Dutzmann, Stefan, Dr.
Leinenweberweg 33
D-4000 Düsseldorf 13(DE)

(54) **Substituierte Pyrimidine.**

(57) Substituierte Pyrimidine der Formel (I)

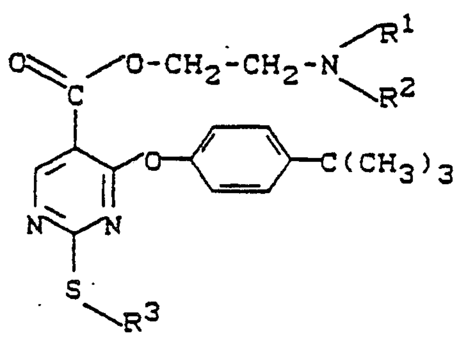

(I)

in welcher
$R^1$, $R^2$ und $R^3$ unabhängig voneinander für Alkyl stehen, und ihre Säureadditionssalze sowie ihre Verwendung in Schädlingsbekämpfungsmitteln.

Die neuen substituierten Pyrimidine sind durch die Formel (I) definiert. Sie können nach Analogieverfahren z.B. aus geeigneten Pyrimidin-5-carbonsäure-Aktivestern und geeigneten Ethanolaminen hergestellt werden. Die Pyrimidin-5-carbonsäure-Aktivester sind ebenfalls neu und können aus den bekannten Carbonsäuren durch Umsetzung mit geeigneten Phenolen erhalten werden.

EP 0 320 775 A2

## Substituierte Pyrimidine

Die Erfindung betrifft neue substituierte Pyrimidine, ein Verfahren zu ihrer Herstellung und ihre Verwendung als Schädlingsbekämpfungsmittel.

Es ist bekannt, daß bestimmte substituierte Pyrimidine, wie beispielsweise die Verbindung 4-(4-t-Butylphenoxy)-2-isobutylthiopyrimidin-5-carbonsäurepiperidid fungizide Eigenschaften besitzen (vgl. DE-OS 33 38 859).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Pyrimidine der allgemeinen Formel (I),

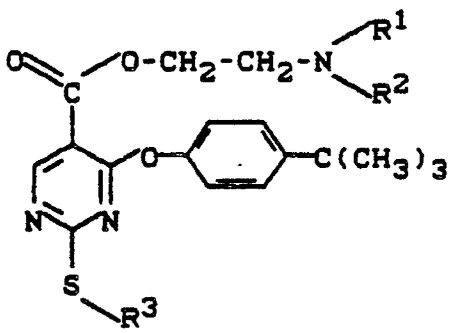

(I)

in welcher
$R^1$ für Alkyl steht,
$R^2$ für Alkyl steht und
$R^3$ für Alkyl steht,
und deren Säureadditionssalze gefunden.

Weiterhin wurde gefunden, daß man die neuen substituierten Pyrimidine der allgemeinen Formel (I),

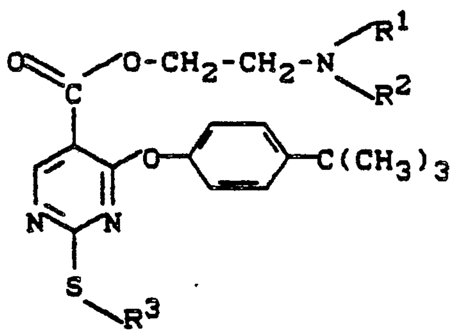

(I)

in welcher
$R^1$ für Alkyl steht,
$R^2$ für Alkyl steht und
$R^3$ für Alkyl steht,
und deren Säureadditionssalze erhält, wenn man Pyrimidin-5-carbonsäure-Aktivester der Formel (II),

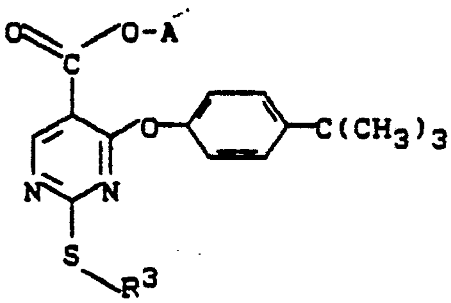

(II)

in welcher
A für eine elektronenanziehende Abgangsgruppe steht und
$R^3$ für Alkyl steht,
mit Ethanolaminen der Formel (III),

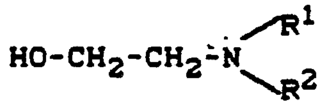

(III)

in welcher
$R^1$ für Alkyl steht und
$R^2$ für Alkyl steht,
gegebenenfalls in Gegenwart eines Verdünnungsmittels umsetzt und gegebenenfalls anschließend eine Säure addiert.

Schließlich wurde gefunden, daß die neuen substituierten Pyrimidine der Formel (I) und deren Säureadditionssalze eine gute Wirkung gegen Schädlinge besitzen.

Überraschenderweise zeigen die erfindungsgemäßen substituierten Pyrimidine der allgemeinen Formel (I) z.B. eine erheblich bessere fungizide Wirksamkeit als die aus dem Stand der Technik bekannten substituierten Pyrimidine, wie beispielsweise die Verbindung 4-(4-t-Butylphenoxy)-2-isobutylthiopyrimidin-5-carbonsäurepiperidid, welches chemisch und wirkungsmäßig naheliegende Verbindungen sind.

Die erfindungsgemäßen substituierten Pyrimidine sind durch die Formel (I) allgemein definiert. Bevorzugt sind Verbindungen der Formel (I), bei welchen
$R^1$, $R^2$ und $R^3$ unabhängig voneinander jeweils für jeweils geradkettiges oder verzweigtes Alkyl mit jeweils 1 bis 4 Kohlenstoffatomen stehen.

Besonders bevorzugt sind Verbindungen, bei welchen
$R^1$ und $R^2$ unabhängig voneinander jeweils für Methyl, Ethyl, n-Propyl oder n-Butyl stehen und
$R^3$ für n-Propyl, i-Propyl, n-Butyl oder i-Butyl steht.

Ganz besonders bevorzugt sind Verbindungen der Formel (I), bei welchen
$R^1$ und $R^2$ für Methyl oder Ethyl stehen und
$R^3$ für n-Propyl oder i-Propyl steht.

Bevorzugte erfindungsgemäße Verbindungen sind auch Additionsprodukte aus Säuren und denjenigen substituierten Pyrimidinen der Formel (I), in denen die Substituenten $R^1$, $R^2$ und $R^3$ die Bedeutungen haben, die bereits vorzugsweise für diese Substituenten genannt wurden.

Zu pflanzenverträglichen Säuren die addiert werden können, gehören vorzugsweise Halogenwasserstoffsäuren, wie z. B. Chlorwasserstoffsäure und Bromwasserstoffsäure, insbesondere Chlorwasserstoffsäure, ferner Phosphorsäure, Salpetersäure, Schwefelsäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z. B. p-Toluolsulfonsäure und 1,5-Naphthalindisulfonsäure, ferner Saccharin oder Thiosaccharin.

Verwendet man beispielsweise 4-(4-t-Butyl-phenoxy)-2-n-propylthio-pyrimidin-5-carbonsäure-(2,4,5-trichlorphenylester) und N-Dimethylethanolamin als Ausgangsstoffe, so läßt sich der Reaktionsablauf des erfindungsgemäßen Verfahrens durch das folgende Formelschema darstellen:

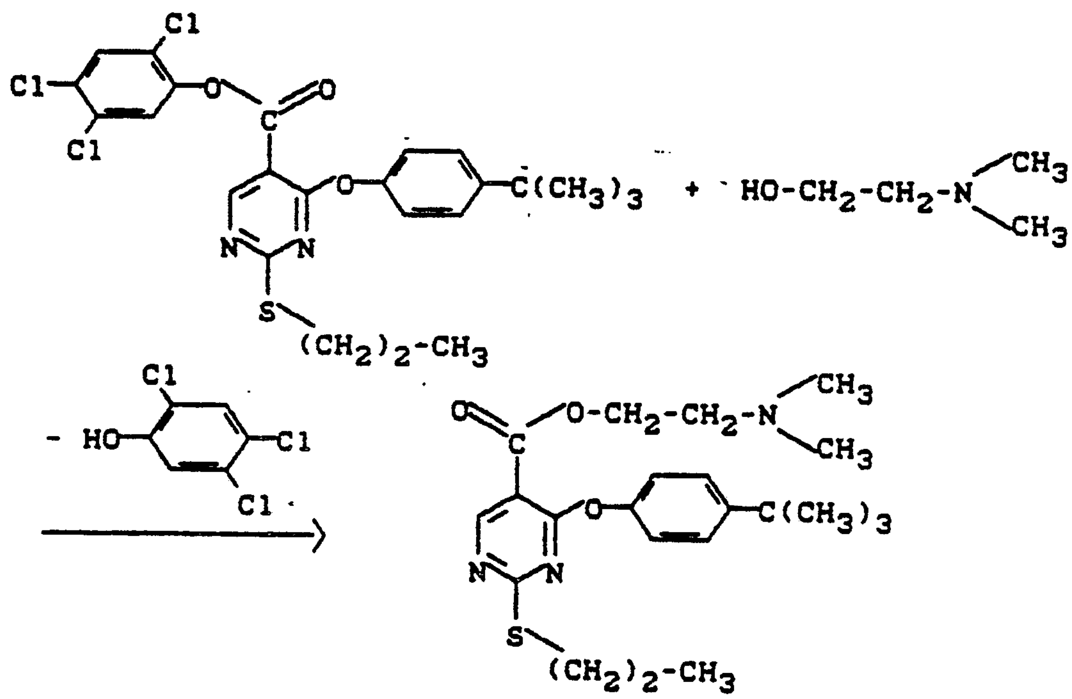

Die zur Durchführung des erfindungsgemäßen Verfahrens als Ausgangsstoffe benötigten Pyrimidin-5-carbonsäure-Aktivester sind durch die Formel (II) allgemein definiert. In dieser Formel (II) steht $R^3$ vorzugsweise für diejenigen Reste, die bereits im Zusammenhang mit der Beschreibung der erfindungsgemäßen Stoffe der Formel (I) als bevorzugt für diesen Substituenten genannt wurden. A steht für einen gebräuchlichen Aktivesterrest, vorzugsweise für einen einfach bis fünffach, gleich oder verschieden substituierten Phenylrest, wobei insbesondere elektronegative Substituenten wie Halogen oder Nitro in Frage kommen.

Die Pyrimidin-5-carbonsäure-Aktivester der Formel (II) sind noch nicht bekannt.

Man erhält sie jedoch in Analogie zu bekannten Verfahren, wenn man die entsprechenden Pyrimidin-5-carbonsäuren der Formel (IV),

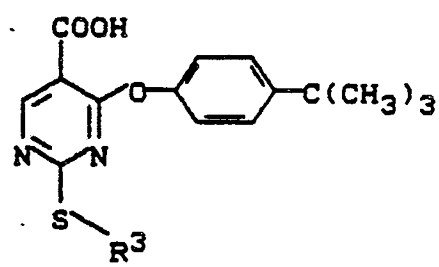

(IV)

in welcher

$R^3$ die oben angegebene Bedeutung hat,

mit Phenolen der Formel (V),

A - OH (V)

in welcher

A die oben angegebene Bedeutung hat,

in Gegenwart eines üblichen Kondensationsmittels, wie beispielsweise N, N′-Dicyclohexylcarbodiimid, sowie gegebenenfalls in Gegenwart eines Verdünnungsmittels, wie beispielsweise Dichlormethan und gegebenenfalls in Gegenwart eines Katalysators, wie beispielsweise 4-(1-(Pyrrolidino)-pyridin, bei Temperaturen zwischen -20 °C und +80 °C kondensiert.

Die Pyrimidin-5-carbonsäuren der Formel (IV) sind bekannt (vgl. DE-OS 33 38 859).

Die Phenole der Formel (V) sind allgemein bekannte Verbindungen der organischen Chemie.

Die zur Durchführung des erfindungsgemäßen Verfahrens als Ausgangsstoffe benötigten Ethanolamine

sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Ethanolamine der Formel (III) sind ebenfalls allgemein bekannte Verbindungen der organischen Chemie.

Als Verdünnungsmittel zur Durchführung des erfindungsgemäßen Verfahrens kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 120 °C, vorzugsweise bei Temperaturen zwischen 20 °C und 80 °C.

Zur Durchführung des erfindungsgemäßen Verfahrens setzt man pro Mol an Pyrimidin-5-carbonsäure-Aktivester der Formel (II) im allgemeinen 1.0 bis 3.0 Mol, vorzugsweise 1.0 bis 1.2 Mol an Ethanolamin der Formel (III) ein. Die Reaktionsdurchführung, Aufarbeitung und Isolierung der Reaktionsprodukte erfolgt nach allgemein üblichen Methoden.

Die Säureadditionssalze der Verbindungen der Formel (I) können in einfacher Weise nach üblichen Salzbildungsmethoden, wie z. B. durch Lösen einer Verbindung der Formel (I) in einem geeigneten inerten Lösungsmittel und Hinzufügen der Säure, wie z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung des echten Getreidemehltaus (Erysiphe graminis) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte, Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethyl sulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wid hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0.0001 bis 0,02 % am Wirkungsort erforderlich.

Herstellungsbeispiele

Beispiel 1

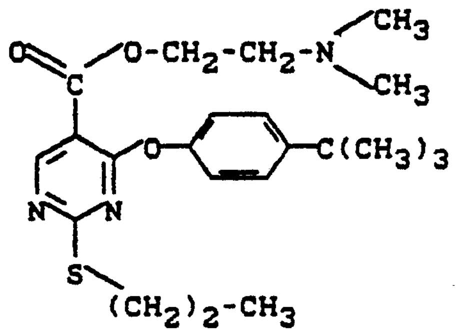

Zu 1,0 g (0.0019 Mol) 4-(4-t-Butylphenoxy)-2-n-propylthiopyrimidin-5-carbonsäure-(2,4,5-trichlorphenylester) in 5 ml absolutem Dimethylformamid gibt man unter Rühren und Feuchtigkeitsausschluß 0,17 g (0.0019 Mol) 2-(N,N-Dimethylamino)-ethanol und rührt anschließend 90 Minuten bei 60 °C und danach 15 Stunden bei Raumtemperatur. Zur Aufarbeitung gibt man Dichlormethan zu der Reaktionsmischung, wäscht zweimal mit Wasser, trocknet über Natriumsulfat, engt im Vakuum ein und reinigt den Rückstand chromatographisch (Kieselgel; Laufmittel: Dichlormethan/Methanol, 10 : 1).

Man erhält 0,7 g (88 % der Theorie) an 4-(4-t-Butylphenoxy)-2-n-propylthiopyrimidin-5-carbonsäure-[2-(N,N-dimethylamino)-ethylester] als Öl.

$^1$H-NMR ($DCCl_3$/Tetramethylsilan):
$\delta$ = 9.0 (s, 1H), 4.4 (t, 2H), 2,35 (s, 6H) ppm.

Herstellung der Ausgangsverbindung

Beispiel (II-1)

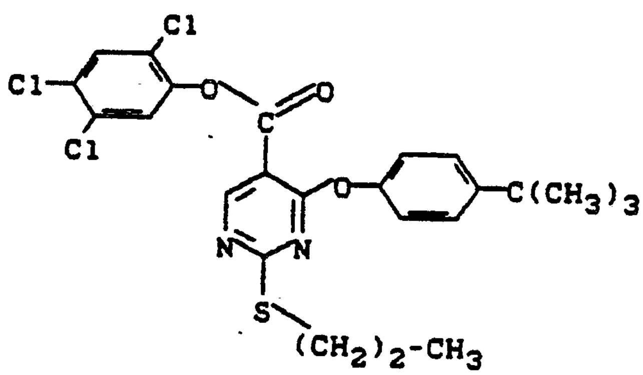

Zu 4,8 g (0.013 Mol) 4-(4-t--Butylphenoxy)-2-n-propylthiopyrimidin-5-carbonsäure (vgl. DE-OS 33 38 859), 3,0 g (0.0152 Mol) 2,4,5-Trichlorphenol und 0,2 g (0,013 Mol) 4-(1-Pyrrolidino)-pyridin in 90 ml Dichlormethan gibt man bei Raumtemperatur unter Rühren und Feuchtigkeitsausschluß 3,2 g (0.0155 Mol) N,N′-Dicyclohexylcarbodiimid und rührt anschließend 16 Stunden bei Raumtemperatur. Zur Aufarbeitung wird filtriert, das Filtrat im Vakuum eingeengt und an Kieselgel chromatographiert (Laufmittel: Dichlormethan/Cyclohexan, 40 : 15).

Man erhält 6,5 g (90 % der Theorie) an 4-(4-t-Butylphenoxy)-2-n-propylthiopyrimidin-5-carbonsäure-(2.4,5-trichlorphenylester) als Öl.

$^1$H-NMR ($CDCl_3$/Tetramethylsilan):
$\delta$ = 9,1 (s, 1H), 7,6 (s, 1H), 7,5 (s, 1H) ppm.

In entsprechender Weise erhält man Beispiel 2:

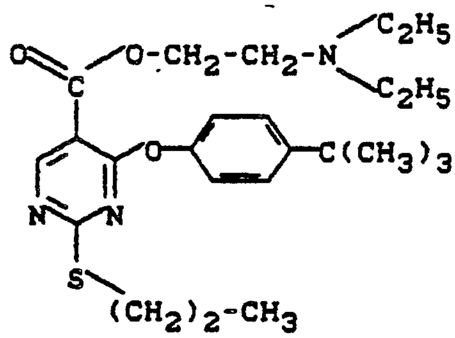

$^1$H-NMR ($CDCl_3$/Tetramethylsilan):
$\delta$ = 8,9 (s, 1H), 4,4 (t, 2H), 1,05 (t, 6H) ppm

Anwendungsbeispiele

In dem folgenden Anwendungsbeispiel wurde die nachstehend aufgeführte Verbindung als Vergleichssubstanz eingesetzt:

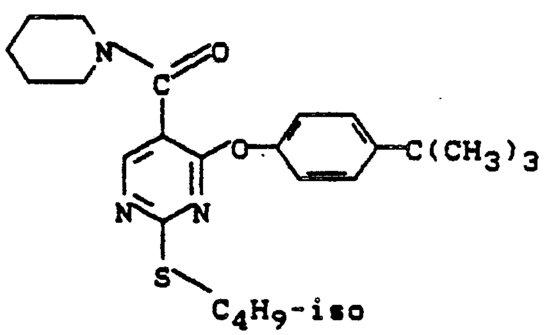

(A)

4-(4-t-Butylphenoxy)-2-isobutylthiopyrimidin-5-carbonsäurepiperidid
(bekannt aus DE-OS 33 38 859).

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß den Herstellungsbeispielen: (1) und (2).

## Ansprüche

1. Substituierte Pyrimidine der allgemeinen Formel (I),

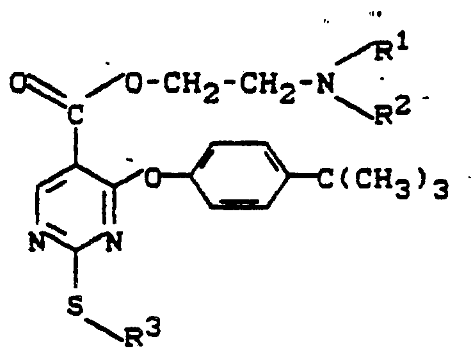

(I)

in welcher
$R^1$ für Alkyl steht,
$R^2$ für Alkyl steht und
$R^3$ für Alkyl steht,
und deren Säureadditionssalze.

2. Substituierte Pyrimidine gemäß Anspruch 1, wobei in der Formel (I)
$R^1$, $R^2$ und $R^3$ unabhängig voneinander jeweils für jeweils geradkettiges oder verzweigtes Alkyl mit jeweils 1 bis 4 Kohlenstoffatomen stehen.

3. Substituierte Pyrimidine gemäß Anspruch 1, wobei in der Formel (I)
$R^1$ und $R^2$ unabhängig voneinander jeweils für Methyl, Ethyl, n-Propyl oder n-Butyl stehen und
$R^3$ für n-Propyl, i-Propyl, n-Butyl oder i-Butyl steht.

4. Substituierte Pyrimidine gemäß Anspruch 1, wobei in der Formel (I)
$R^1$ und $R^2$ für Methyl oder Ethyl stehen und
$R^3$ für n-Propyl oder i-Propyl steht.

5. Verfahren zur Herstellung von substituierten Pyrimidinen der Formel (I),

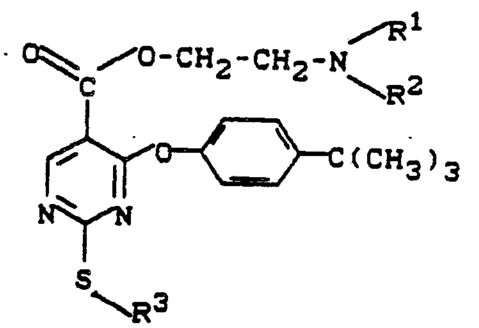

(I)

in welcher
$R^1$ für Alkyl steht,
$R^2$ für Alkyl steht und
$R^3$ für Alkyl steht,
und deren Säureadditionssalze, dadurch gekennzeichnet, daß man
Pyrimidin-5-carbonsäure-Aktivester der Formel (II),

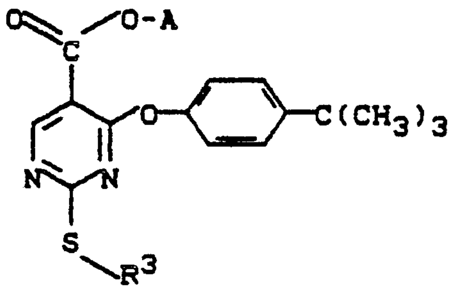

(II)

in welcher
A für eine elektronenanziehende Abgangsgruppe steht und
$R^3$ für Alkyl steht,
mit Ethanolaminen der Formel (III),

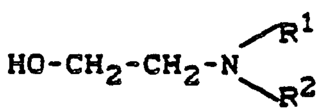

(III)

in welcher
$R^1$ für Alkyl steht und
$R^2$ für Alkyl steht,
gegebenenfalls in Gegenwart eines Verdünnungsmittels umsetzt und gegebenenfalls anschließend eine Säure addiert.

6. Schädlingsbekämpfungsmittel, gekennzeichnet durch einen Gehalt an mindestens einem substituierten Pyrimidin der Formel (I) nach den Ansprüchen 1 und 5.

7. Verwendung von substituierten Pyrimidinen der Formel (I) nach den Ansprüchen 1 und 5 zur Bekämpfung von Schädlingen.

8. Verfahren zur Bekämpfung von Schädlingen, dadurch gekennzeichnet, daß man substituierte Pyrimidine der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man substituierte Pyrimidine der Formel (I) nach Ansprüchen 1 und 5 mit Streckmitteln und/oder oberflächenaktiven Mitteln vermischt.

10. Pyrimidin-5-carbonsäure-Aktivester der Formel (II),

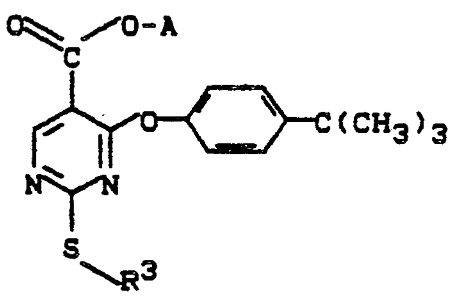

(II)

in welcher
A für eine elektronenanziehende Abgangsgruppe steht und
$R^3$ für Alkyl steht.

11. Verfahren zur Herstellung von Pyrimidin-5-carbonsäure-Aktivestern der Formel (II),

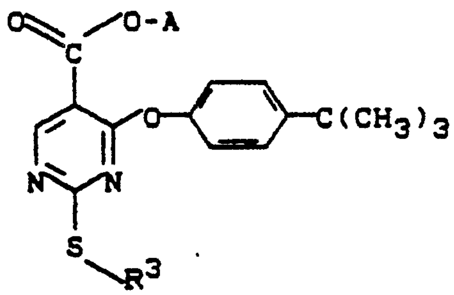

(II)

in welcher
A für eine elektronenanziehende Abgangsgruppe steht und
$R^3$ für Alkyl steht, dadurch gekennzeichnet, daß man
Pyrimidin-5-carbonsäuren der Formel (IV),

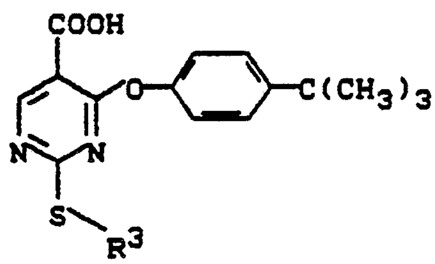

(IV)

in welcher
$R^3$ die oben angegebene Bedeutung hat,
mit Phenolen der Formel (V),
A - OH (V)
in welcher
A die oben angegebene Bedeutung hat,
in Gegenwart eines üblichen Kondensationsmittels, gegebenenfalls in Gegenwart eines Verdünnungsmittels und gegebenenfalls in Gegenwart eines Katalysators bei Temperaturen zwischen -20 °C und +80 °C kondensiert.

12. Pyrimidin-5-carbonsäure-Aktivester der Formel (II) gemäß den Ansprüchen 10 und 11, in welcher A für einen gegebenenfalls ein- bis fünffach, gleich oder verschieden durch Halogen und Nitro substituierten Phenylrest steht.